# EUROPEAN PATENT SPECIFICATION

(11) **EP 1 605 964 B1**
(45) Date of publication and mention of the grant of the patent: **10.03.2010**
(21) Application number: 03767704.4
(22) Date of filing: 28.11.2003
(51) Int. Cl.: A61K 38/17, C12N 15/09, C12N 15/12, C07K 14/47, A61P 35/00, A61P 25/28, A61P 29/00, A61P 17/06, A61P 9/00

(54) **TARGETED PROTEIN DEGRADATION VEHICLES (TPDVs), NUCLEIC ACID CONSTRUCTS ENCODING THEM AND THEIR USE**
GEZIELTE PROTEINE ABBAUTRÄGER, DAFÜR KODIERENDE NUKLEINSAÜRE UND DEREN VERWENDUNG
VEHICULES DE DEGRADATION DE PROTEINES CIBLES, CONSTRUCTIONS D'ACIDES NUCLEIQUES CODANT POUR CEUX-CI ET LEUR UTILISATION

(30) Priority: 29.11.2002 EP 02026850
(43) Date of publication of application: 21.12.2005
(73) Proprietor: GEORG-SPEYER-HAUS CHEMOTHERAPEUTISCHES FORSCHUNGSINSTITUT, D-60596 Frankfurt (DE)
(72) Inventor: BÜRGER, Claudia, Toronto, ON M5T 2N2 (CA); GRONER, Bernd, 60322 Frankfurt/Main (DE); NAGEL-WOLFRUM, Kerstin, 55124 Mainz (DE); KUNZ, Christian, 60326 Frankfurt/Main (DE); WITTIG, Ilka, 60529 Frankfurt/Main (DE)
(74) Representative: Kalhammer, Georg
(86) International application number: PCT/EP2003/013436
(87) International publication number: WO 2004/050113

(56) References cited:
- EP-A- 1 205 191
- WO-A-00/37636
- WO-A-01/75453
- WO-A-01/83518
- XIA LING ET AL: "Identification of both positive and negative domains within the epidermal growth factor receptor COOH-terminal region for signal transducer and activator of transcription (STAT) activation." JOURNAL OF BIOLOGICAL CHEMISTRY, vol. 277, no. 34, 23 August 2002 (2002-08-23), pages 30716-30723, XP002240717 August 23, 2002 ISSN: 0021-9258
- KAMIZONO SHINTARO ET AL: "The SOCS box of SOCS-1 accelerates ubiquitin-dependent proteolysis of TEL-JAK2." JOURNAL OF BIOLOGICAL CHEMISTRY, vol. 276, no. 16, 20 April 2001 (2001-04-20), pages 12530-12538, XP002240718 ISSN: 0021-9258

### SEQUENCE LISTING

<110> Georg Speyer Haus Chemotherapeutisches Forschungsinstitut
<120> Targeted protein degradation vehicles (TPDVS), nucleic acid constructs encoding them and their use
<130> TPDV
<150> EP 02026850.4
   <151> 2002-11-29
<160> 23
<170> PatentIn version 3.1
<210> 1
   <211> 20
   <212> PRT
   <213> Artificial Sequence
<220>
   <223> KDI1
<400> 1
   Val Phe Gly Val Ser Trp Val Val Gly Phe Trp Cys Gln Met His Arg
   1               5                   10                  15
   Arg Leu Val Cys
               20
<210> 2
   <211> 20
   <212> PRT
   <213> Artificial Sequence
<220>
   <223> KDI3
<400> 2
   Thr Phe Ser Pro Val Val Leu Leu Tyr Arg Leu Phe Leu Ala Thr Val
   1               5                   10                  15
   Leu Ile Trp Ala
               20
<210> 3
   <211> 20
   <212> PRT
   <213> Artificial Sequence
<220>
   <223> KDI4
<400> 3
   Leu Ser Phe Gln Leu Ser Pro Leu Ser Leu Val His Arg Trp Gly Thr
   1               5                   10                  15
   Thr Val Asp Val
               20
<210> 4
   <211> 42
   <212> PRT
   <213> Artificial sequence
<220>
   <223> 3.40
<400> 4
   Ser Pro Ile Ser Ile Pro Ile Gly Phe Val Val Arg His Cys Ala Leu
   1               5                   10                  15
   His Met Ala Val Gly Pro Leu Ser Trp Pro Ala Arg Val Ser Gly Tyr
               20                  25                  30
   Ser Phe Ala Leu Glu Val Leu Thr Asn Phe
           35                  40
<210> 5
   <211> 11
   <212> PRT
   <213> Artificial Sequence
<220>
   <223> HIV-1 Tat
<400> 5
   Tyr Gly Arg Lys Lys Arg Arg Gln Arg Arg Arg
   1               5                   10
<210> 6
   <211> 34
   <212> PRT
   <213> Artificial sequence
<220>
   <223> HSV VP22
<400> 6
   Asp Ala Ala Thr Ala Thr Arg Gly Arg Ser Ala Ala Ser Arg Pro Thr
   1               5                   10                  15
   Glu Arg Pro Arg Ala Pro Ala Arg Ser Ala Ser Arg Pro Arg Arg Pro
               20                  25                  30
   Val Glu
<210> 7
   <211> 16
   <212> PRT
   <213> Artificial Sequence
<220>
   <223> ANTP
<400> 7
   Arg Gln Ile Lys Ile Trp Phe Gln Asn Arg Arg Met Lys Trp Lys Lys
   1 5 10 15
<210> 8
   <211> 765
   <212> DNA
   <213> Artificial Sequence
<220>
   <223> pET KDI1-9RSOCS
<400> 8
   atgcaccatc atcatcatca ttcttctggt ctggtgccac gcggttctgg tatgaaagaa 60
   accgctgctg ctaaattcga acgccagcac atggacagcc cagatctggg taccgacgac 120
   gacgacaagg ccatggctga tatcggatcc gaattccata tgagcgataa aattattcac 180
   ctgactgacg acagttttga cacggatccg gatgtactca aagcggacgg ggcgatcctc 240
   gtcgatttct gggcagagtg gtgcggtccg gtgtttgggg tgtcttgggt ggttgggttt 300
   tggtgtcaga tgcatcggcg gcttgtttgt ggtccgtgca aaatgatcgc cccgattctg 360
   gatgaaatcg ctgacgaata tcagggcaaa ctgaccgttg caaaactgaa catcgatcaa 420
   aaccctggca ctgcgccgaa atatggcatc cgtggtatcc cgactctgct gctgttcaaa 480
   aacggtgaag tggcggcaac caaagtgggt gcactgtcta aaggtcagtt gaaagagttc 540
   ctcgacgcta acctggccga attcgggcgt cgtcgtcgtc gccgtcgtcg gcggccgcat 600
   atggtcgacc cgctgcgcca gcgccgcgtg cggccgctgc aggagctgtg ccgccagcgc 660
   atcgtggcca ccgtgggccg cgagaacctg gctcgcatcc ccctcaaccc cgtcctccgc 720
   gactacctga gctccttccc cctcgagcac caccaccacc accac 765
<210> 9
   <211> 765
   <212> DNA
   <213> Artificial sequence
<220>
   <223> pET30 KDI3-9RSOC5
<400> 9
   atgcaccatc atcatcatca ttcttctggt ctggtgccac gcggttctgg tatgaaagaa 60
   accgctgctg ctaaattcga acgccagcac atggacagcc cagatctggg taccgacgac 120
   gacgacaagg ccatggctga tatcggatcc gaattccata tgagcgataa aattattcac 180
   ctgactgacg acagttttga cacggatccg gatgtactca aagcggacgg ggcgatcctc 240
   gtcgatttct gggcagagtg gtgcggtccg actttttctc ctgttgtttt gctgtatcgt 300
   ttgtttctgg cgacggtgtt gatttgggct ggtccgtgca aaatgatcgc cccgattctg 360
   gatgaaatcg ctgacgaata tcagggcaaa ctgaccgttg caaaactgaa catcgatcaa 420
   aaccctggca ctgcgccgaa atatggcatc cgtggtatcc cgactctgct gctgttcaaa 480
   aacggtgaag tggcggcaac caaagtgggt gcactgtcta aaggtcagtt gaaagagttc 540
   ctcgacgcta acctggccga attcgggcgt cgtcgtcgtc gccgtcgtcg gcggccgcat 600
   atggtcgacc cgctgcgcca gcgccgcgtg cggccgctgc aggagctgtg ccgccagcgc 660
   atcgtggcca ccgtgggccg cgagaacctg gctcgcatcc ccctcaaccc cgtcctccgc 720
   gactacctga gctccttccc cctcgagcac caccaccacc accac 765
<210> 10
   <211> 765
   <212> DNA
   <213> Artificial Sequence
<220>
   <223> pET30 KDI4-9RSOCS
<400> 10
   atgcaccatc atcatcatca ttcttctggt ctggtgccac gcggttctgg tatgaaagaa 60
   accgctgctg ctaaattcga acgccagcac atggacagcc cagatctggg taccgacgac 120
   gacgacaagg ccatggctga tatcggatcc gaattccata tgagcgataa aattattcac 180
   ctgactgacg acagttttga cacggatccg gatgtactca aagcggacgg ggcgatcctc 240
   gtcgatttct gggcagagtg gtgcggtccg ttgtcttttc agttgtctcc gctgtcgttg 300
   gtgcatcggt gggggacgac tgttgatgtg ggtccgtgca aaatgatcgc cccgattctg 360
   gatgaaatcg ctgacgaata tcagggcaaa ctgaccgttg caaaactgaa catcgatcaa 420
   aaccctggca ctgcgccgaa atatggcatc cgtggtatcc cgactctgct gctgttcaaa 480
   aacggtgaag tggcggcaac caaagtgggt gcactgtcta aaggtcagtt gaaagagttc 540
   ctcgacgcta acctggccga attcgggcgt cgtcgtcgtc gccgtcgtcg gcggccgcat 600
   atggtcgacc cgctgcgcca gcgccgcgtg cggccgctgc aggagctgtg ccgccagcgc 660
   atcgtggcca ccgtgggccg cgagaacctg gctcgcatcc ccctcaaccc cgtcctccgc 720
   gactacctga gctccttccc cctcgagcac caccaccacc accac 765
<210> 11
   <211> 830
   <212> DNA
   <213> Artificial Sequence
<220>
   <223> pET30 STAT3.40-9RSOCS
<400> 11
   atgcaccatc atcatcatca ttcttctggt ctggtgccac gcggttctgg tatgaaagaa 60
   accgctgctg ctaaattcga acgccagcac atggacagcc cagatctggg taccgacgac 120
   gacgacaagg ccatggctga tatcggatcc gaattccata tgagcgataa aattattcac 180
   ctgactgacg acagttttga cacggatccg gatgtactca aagcggacgg ggcgatcctc 240
   gtcgatttct gggcagagtg gtgcggtccg tctcctatta gtattccgat tgggtttgtt 300
   gttcggcatt gtgcgcttca tatggcggtg ggtccgttgt cttggccggc gagggtttct 360
   gggtattcgt ttgctttgga ggtgctgact aattttggtc cgtgcaaaat gatcgccccg 420
   attctggatg aaatcgctga cgaatatcag ggcaaactga ccgttgcaaa actgaacatc 480
   gatcaaaacc ctggcactgc gccgaaatat ggcatccgtg gtatcccgac tctgctgctg 540
   ttcaaaaacg gtgaagtggc ggcaaccaaa gtgggtgcac tgtctaaagg tcagttgaaa 600
   gagttcctcg acgctaacct ggccgaattc gggcgtcgtc gtcgtcgccg tcgtcggcgg 660
   ccgcatatgg tcgacccgct gcgccagcgc cgcgtgcggc cgctgcagga gctgtgccgc 720
   cagcgcatcg tggccaccgt gggccgcgag aacctggctc gcatccccct caaccccgtc 780
   cccgcgacta cctgagctcc ttccccctcg agcaccacca ccaccaccac 830
<210> 12
   <211> 35
   <212> PRT
   <213> Artificial Sequence
<220>
   <223> SOCS-box
<400> 12
   Pro Leu Gln Glu Leu Cys Arg Gln Arg Ile Val Ala Thr Val Gly Arg
   1 5 10 15
   Glu Asn Leu Ala Arg Ile Pro Leu Asn Pro Val Leu Arg Asp Tyr Leu
   20 25 30
   Ser Ser Phe
   35
<210> 13
   <211> 50
   <212> PRT
   <213> Artificial Sequence
<220>
   <223> F-box
<400> 13
   Lys Pro Phe Pro Leu Leu Arg Leu Pro Glu Glu Ile Leu Arg Lys Ile
   1 5 10 15
   Leu Glu Lys Leu Asp Pro Ile Asp Leu Leu Arg Leu Arg Lys Val Ser
   20 25 30
   Lys Lys Trp Arg Ser Leu Val Asp Ser Leu Asn Ile Trp Phe Lys Phe
   35 40 45
   Ile Glu
   50
<210> 14
   <211> 100
   <212> PRT
   <213> Artificial Sequence
<220>
   <223> HECT domain
   <400> 14
   Tyr Asp Gly Gly Tyr Thr Arg Asp Ser Val Leu Ile Arg Glu Phe Trp
   1 5 10 15
   Glu Ile Val His Ser Phe Thr Asp Glu Gln Lys Arg Leu Phe Leu Gln
   20 25 30
   Phe Thr Thr Gly Thr Asp Arg Ala Pro Val Gly Gly Leu Gly Lys Leu
   35 40 45
   Lys Met Ile Ile Ala Lys Asn Gly Pro Asp Thr Glu Arg Leu Pro Thr
   50 55 60
   Ser His Thr Cys Phe Asn Val Leu Leu Leu Pro Glu Tyr Ser Ser Lys
   65 70 75 80
   Glu Lys Leu Lys Glu Arg Leu Leu Lys Ala Ile Thr Tyr Ala Lys Gly
   85 90 95
   Phe Gly Met Leu
   100
<210> 15
   <211> 70
   <212> PRT
   <213> Artificial sequence
<220>
   <223> U-box
<400> 15
   Asp Tyr Ser Asp Ala Pro Asp Glu Phe Arg Asp Pro Leu Met Asp Thr
   1 5 10 15
   Leu Met Thr Asp Pro Val Arg Leu Pro Ser Gly Thr Val Met Asp Arg
   20 25 30
   Ser Ile Ile Leu Arg His Leu Leu Asn Ser Pro Thr Asp Pro Phe Asn
   35 40 45
   Arg Gln Met Leu Thr Glu Ser Met Leu Glu Pro Val Pro Glu Leu Lys
   50 55 60
   Glu Gln Ile Gln Ala Trp
   65 70
<210> 16
   <211> 42
   <212> PRT
   <213> artificial sequence
<220>
   <223> DD-1
<400> 16
   Pro Pro Leu Val Cys Ile Arg Ser Trp Cys Pro Leu Met Val Pro His
   1 5 10 15
   Ser Ala Asp Leu Gly Pro Ala Ser Gln Trp Leu Cys His Arg Val Ala
   20 25 30
   Ser Ile Ala Leu Leu Pro Arg Tyr Ser Ser
   35 40
<210> 17
   <211> 42
   <212> PRT
   <213> artificial sequence
<220>
   <223> DD-2
<400> 17
   Val Gly Trp Thr Trp Met Ser Val Leu Val Cys Cys Asp Gly Ser Phe
   1 5 10 15
   Leu Val Pro Glu Gly Pro Val Val Val Gln Ala Gly Gly Ala Val Pro
   20 25 30
   Ile Ser Gly Ser Val Ala Leu Met Thr Asp
   35 40
<210> 18
   <211> 19
   <212> PRT
   <213> artificial sequence
<220>
   <223> DBD-1
<400> 18
   Pro Leu Thr Ala Val Phe Trp Leu Ile Tyr Val Leu Ala Lys Ala Leu
   1 5 10 15
   Val Thr Val
<210> 19
   <211> 9
   <212> PRT
   <213> artificial sequence
<220>
   <223> 9R
<400> 19
   Arg Arg Arg Arg Arg Arg Arg Arg Arg
   1 5
<210> 20
   <211> 9
   <212> PRT
   <213> artificial sequence
<220>
   <223> 9K
<400> 20
   Lys Lys Lys Lys Lys Lys Lys Lys Lys
   1 5
<210> 21
   <211> 825
   <212> DNA
   <213> Artificial sequence
<220>
   <223> pET 30 DD-1-9RSOC5
<400> 21
   atgcaccatc atcatcatca ttcttctggt ctggtgccac gcggttctgg tatgaaagaa 60
   accgctgctg ctaaattcga acgccagcac atggacagcc cagatctggg taccgacgac 120
   gacgacaagg ccatggctga tatcggatcc gaattccata tgagcgataa aattattcac 180
   ctgactgacg acagttttga cacggatgta ctcaaagcgg acggggcgat cctcgtcgat 240
   ttctgggcag agtggtgcgg tccgccgccg ctggtttgta ttaggtcttg gtgtcctttg 300
   atggtgccgc attctgctga tttgggtccg gcgtcgcagt ggctgtgtca tcgtgttgct 360
   tctattgctt tgttgccgcg ttattcgtct ggtccgtgca aaatgatcgc cccgattctg 420
   gatgaaatcg ctgacgaata tcagggcaaa ctgaccgttg caaaactgaa catcgatcaa 480
   aaccctggca ctgcgccgaa atatggcatc cgtggtatcc cgactctgct gctgttcaaa 540
   aacggtgaag tggcggcaac caaagtgggt gcactgtcta aaggtcagtt gaaagagttc 600
   ctcgacgcta acctggccga attcgggcgt cgtcgtcgtc gccgtcgtcg gcggccgcat 660
   atggtcgacc cgctgcgcca gcgccgcgtg cggccgctgc aggagctgtg ccgccagcgc 720
   atcgtggcca ccgtgggccg cgagaacctg gctcgcatcc ccctcaaccc cgtcctccgc 780
   gactacctga gctccttccc cctcgagcac caccaccacc accac 825
<210> 22
   <211> 825
   <212> DNA
   <213> Artificial Sequence
<220>
   <223> pET 30 DD-2-9RSOCS
<400> 22
   atgcaccatc atcatcatca ttcttctggt ctggtgccac gcggttctgg tatgaaagaa 60
   accgctgctg ctaaattcga acgccagcac atggacagcc cagatctggg taccgacgac 120
   gacgacaagg ccatggctga tatcggatcc gaattccata tgagcgataa aattattcac 180
   ctgactgacg acagttttga cacggatgta ctcaaagcgg acggggcgat cctcgtcgat 240
   ttctgggcag agtggtgcgg tccggttggt tggacttgga tgtcggtttt ggtttgttgt 300
   gatgggtctt ttttggttcc ggagggtccg gtggttgttc aggcgggtgg ggcggtgcct 360
   attagtggtt cggttgcgtt gatgactgat ggtccgtgca aaatgatcgc cccgattctg 420
   gatgaaatcg ctgacgaata tcagggcaaa ctgaccgttg caaaactgaa catcgatcaa 480
   aaccctggca ctgcgccgaa atatggcatc cgtggtatcc cgactctgct gctgttcaaa 540
   aacggtgaag tggcggcaac caaagtgggt gcactgtcta aaggtcagtt gaaagagttc 600
   ctcgacgcta acctggccga attcgggcgt cgtcgtcgtc gccgtcgtcg gcggccgcat 660
   atggtcgacc cgctgcgcca gcgccgcgtg cggccgctgc aggagctgtg ccgccagcgc 720
   atcgtggcca ccgtgggccg cgagaacctg gctcgcatcc ccctcaaccc cgtcctccgc 780
   gactacctga gctccttccc cctcgagcac caccaccacc accac 825
<210> 23
   <211> 759
   <212> DNA
   <213> Artificial sequence
<220>
   <223> pET 30 DBD-1-9RSOCS
<400> 23
   atgcaccatc atcatcatca ttcttctggt ctggtgccac gcggttctgg tatgaaagaa 60
   accgctgctg ctaaattcga acgccagcac atggacagcc cagatctggg taccgacgac 120
   gacgacaagg ccatggctga tatcggatcc gaattccata tgagcgataa aattattcac 180
   ctgactgacg acagttttga cacggatgta ctcaaagcgg acggggcgat cctcgtcgat 240
   ttctgggcag agtggtgcgg tccgccgttg actgcggtgt tttggttgat ttatgtgttg 300
   gctaaggcgc tggttactgt gtgtggtccg tgcaaaatga tcgccccgat tctggatgaa 360
   atcgctgacg aatatcaggg caaactgacc gttgcaaaac tgaacatcga tcaaaaccct 420
   ggcactgcgc cgaaatatgg catccgtggt atcccgactc tgctgctgtt caaaaacggt 480
   gaagtggcgg caaccaaagt gggtgcactg tctaaaggtc agttgaaaga gttcctcgac 540
   gctaacctgg ccgaattcgg gcgtcgtcgt cgtcgccgtc gtcggcggcc gcatatggtc 600
   gacccgctgc gccagcgccg cgtgcggccg ctgcaggagc tgtgccgcca gcgcatcgtg 660
   gccaccgtgg gccgcgagaa cctggctcgc atccccctca accccgtcct ccgcgactac 720
   ctgagctcct tccccctcga gcaccaccac caccaccac 759

## Description

The present invention relates to peptide TPDV (targeted protein degradation vehicle) tools and their use for therapeutic purposes in the treatment of patients, in particular tumor patients. These vehicles are used for the selected degradation of intracellular target proteins, in particular of oncoproteins, which are involved in tumorigenesis. The invention also relates to nucleic acids coding for TPDVs and to the production of a medicament based on such TPDV drugs.

Despite the tremendous success of medicine there are still various diseases which are difficult to treat. In particular several types of tumors are not curable with known means. It is therefore an object of the present invention to provide alternative means for the treatment of such diseases, in particular various types of cancer. The present invention provides therefore a targeted protein degradation vehicle as defined in the claims.

The targeted protein degradation vehicle according to the present invention comprises at least one affinity domain which allows the binding of the vehicle to the preselected target molecule. An affinity domain is a peptide or polypeptide which specifically binds to a target molecule. Specific binding may be confirmed by interaction in the yeast two-hybrid system (see infra).

### The epidermal growth factor receptor and its role in tumorigenesis

The EGFR family of receptor tyrosine kinases, consisting of the epidermal growth factor (EGF) receptor EGFR, ErbB2/Neu, ErbB3 and ErbB4, binds a large group of ligands (Riese and Stern, 1998, Bioessays, (20) 41-8). The EGF receptor belongs to the group of enzyme-linked receptors which are transmembrane proteins with their ligand-domain on the outer surface of the plasma membrane. On the cytosol site of the cell the EGF receptor has a tyrosine kinase which phosphorylates specific tyrosines on a small set of intracellular signaling proteins. When the signaling ligand epidermal growth factor (EGF) is bound to the EGF receptor the proliferation of various cell types is stimulated.

Upon binding of these ligands to the extracellular domain, the receptor dimerizes, which leads to autophosphorylation on specific tyrosine residues in their cytoplasmic tail. These residues serve as docking sites for SH2- and PTB domain containing signaling molecules, which activate numerous intracellular signaling pathways. A major signaling route of the EGFR family seems to be the ras/raf-mitogen-activated protein kinase (MAPK) pathway (Olayioye, et al., 2000, Embo J, (19) 3159-3167).

EGF can also activate the STAT3 pathway. There is a set of latent gene regulatory proteins called STATs (signal transducers and activators of transcription) which move into the nucleus and stimulate the transcription of specific genes. All STATs have an SH2 domain that enables them to dock onto specific phosphotyrosines on some activated receptor tyrosine kinase receptors. After EGF stimulation the cytoplasmic kinase c-src is recruited to the receptor, where it phosphorylates and thereby hyper-activates the EGFR itself and other signaling molecules as Jak2, STAT3 (Olayioye, et al., 2001, Exp Cell Res, (267) 81-7.) (Olayioye, et al., 1999, J Biol Chem, (274) 17209-18), Shc and components of the cytoskeleton and the endocytic machinery (Wilde, et al., 1999, Cell, (96) 677-87.). Thus, EGFR signaling can lead to a variety of downstream reactions, which results in proliferation, differentiation and/or oncogenesis of epithelial cells, neuronal cells and fibroblasts (Hynes, 2000, Breast Cancer Res, (2) 154-7). The expression of EGFR in tumor cells can be correlated with poor response to treatment, disease progression, and poor survival (Baselga, 2002, Oncologist, (7) 2-8.).

In a preferred embodiment the affinity domain binds specifically to the EGF receptor. It is possible that the affinity domain binds to that part of the EGF receptor which is usually localized on the outer surface of the plasma membrane (extracellular part). It is, however, preferred that the affinity domain binds to a region of the EGFR which is on the cytosolic side of the cell (intracellular part).

In another embodiment, the affinity domain specifically binds to STAT3 protein.

### The signal transducer and activator of transcription STAT3 and its role in tumorigenesis

Signal transducers and activators of transcription (STATs) were originally discovered as mediators of cytokine signaling. Upon binding of cytokines to cognate receptors, receptors dimerize and thereby activate intrinsic receptor tyrosine kinases or receptor associated tyrosine kinases (e.g. JAKs). Tyrosine-phosphorylated receptors provide docking sites for the recruitment of cytoplasmic monomeric STAT proteins via their SH2 domains. Receptor-bound STATs become tyrosine phosphorylated and dimerize via reciprocal phosphotyrosine-SH2 domain interaction. Within minutes, the dimers translocate to the nucleus where they act with other transcriptional modulators bound to specific promoter sequences and induce gene expression (Ihle, 2001, Curr. Opin. Cell Biol (13) 211-17; Buettner, Mora, 2002, Clin.Cancer Res. (8) 945-54). In the last few years, constitutive activation of STAT proteins, e.g. STAT3 and STAT5, have been found in a large number of several human cancers, including blood malignancies (leukemias, lymphomas, and multiple myeloma) as well as solid tissues (such as head and neck, breast, and prostate cancer) (Bowman, Garcia, 2000, Oncogene, (19) 2474-88; Song, Grandis, 2000, Oncogene (19) 2489-95; Coffer, Koedermann, 2000, Oncogene (19) 2511-22; Lin, Mahajan, 2000, Oncogene (19) 2496-2504; Garcia, Bowman, 2001, Oncogene (20) 2499-2513). Studies to date provide strong evidence that aberrant STAT3 signaling participate in the development and progression of human cancer by either preventing apoptosis, inducing cell proliferation, or both (Bowman, Garcia, 2000, Oncogene (19) 2474-88). *In vitro* and *in vivo* inhibition of constitutive STAT3 signaling in tumor cells leads to growth arrest and tumor regression in association with induction of apoptosis, indicating that STAT3 could be a potential target for cancer therapy (Catlett-Falcone, Landowski, 1999, Immunity (10) 105-15; Nui, Shain, 2001 Cancer Res (61) 3276-80; Niu, Heller, 1999, Cancer Res (59) 5050-63).

High levels of EGF receptors and constitutive activation of STAT3 in an appreciable subset of cancer types correlates with an unfavorable prognosis and higher incidence of metastasis. As conventional tumor therapy often results in severe side effects due to high toxicity, new, selective anticancer agents are disclosed that differentiate between malignant and non-malignant cells. Although expressed in non-malignant cells, the high expression levels in a certain subset of tumors, makes the EGFR a perfect target for a specific and selective tumor therapy. The evidence that inhibition of the EGFR and STAT3 can positively influence the pathogenesis of various cancers has led to the present agents that selectively target this molecules (Baselga, 2002, Oncologist, (7) 2-8.) (Turkson and Jove, 2000, Oncogene, (19) 6613-26.).

Since the targeted protein degradation vehicle of the present invention acts specifically on certain target cells it has an affinity domain which binds to a specific target or more precisely to a specific part of this target.

Although a variety of strategies have been suggested for targeting these proteins for an efficient cancer therapy, including monoclonal antibodies, ligand-linked immunotoxins, tyrosine kinase inhibitors, and antisense approaches, the present invention uses in a preferred embodiment peptide aptamers as affinity domain.

### Peptide Aptamers

Peptide aptamers are molecules in which a variable peptide domain with affinity for a given target molecule is displayed from a scaffold protein, such as *E. coli* thioredoxin A. Peptide aptamers present a novel class of molecules, which have a great potential to be applied for the disruption of protein-protein interactions. They can be selected from combinatorial libraries that display conformationally constrained variable regions, which are selected for *in vivo* binding for a given target protein (Colas, 2000, Curr Opin Chem Biol, (4) 54-9). Up to now there are numerous examples that peptide aptamers can disrupt specific protein-protein interactions *in vivo*, which can be used as dominant genetic agents to cause a phenotype (Cohen, et al., 1998, Proc Natl Acad Sci U S A, (95) 14272-7), (Kolonin and Finley, 1998, Proc Natl Acad Sci U S A, (95) 14266-71), (Norman, et al., 1999, Science, (285) 591-5), (Butz, et al., 2001, Oncogene, (20) 6579-86., Butz, et al., 2000, Proc Natl Acad Sci U S A, (97) 6693-7). Other proteins that have been described as scaffolds suitable for the presentation of constrained peptides include a catalytically inactive derivative of the staphylococcal nuclease, the protease inhibitor eglin C, the *Streptomyces tendea* α-amylase inhibitor tendamistat, the cellular transcription factor Sp1, and the green fluorescent protein.

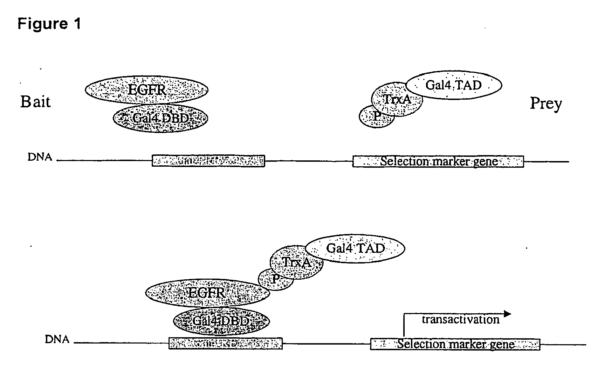

The preferred aptamer selection system is shown schematically in Fig. 1. It is based on a classical yeast-two-hybrid screening system, where upon interaction between the gene of interest and a peptide aptamer, which is inserted into the active loop of the scaffold protein thioredoxin A, a complete Gal4 transcription factor is restored, which allows growth in the presence of certain selection marker.

As aptamers are able to specifically inhibit the functions of cellular proteins, they possess a high potential to be used as therapeutic components. Three strategies are feasible in order to deliver these proteins into target cells (Hoppe-Seyler and Butz, 2000, J Mol Med, (78) 426-30): First, the coding sequence for an aptamer could be introduced into cells, for example by suitable gene therapy vectors, thereby allowing the intracellular expression of specific inhibitors of a given target protein, However, the development of safe and efficient gene delivery systems still represents a major challenge for gene therapy.

Second, one could envision using the peptide aptamers directly as drugs. However, at present time there are still many obstacles to overcome in using proteinaous molecules as drugs. These include the metabolic instability of peptides and proteins, the inability of these molecules to cross cellular membranes, and their potential immunogenicity. However, much progress has been made in this field. A multitude of delivery systems (such as encapsulation of proteins into polymers or lipids) that protect therapeutic proteins from degradation and allow their controlled release have been developed (Langer, 1998, Nature, (392) 5-10.). Peptides and proteins can be stabilized by chemical modifications, for example by modifying chemical bonds and altering amino acid side chains. Shuttle proteins have been identified that can mediate efficient transfer of fused heterologous proteins into cultured cells and even into whole organs in animal systems. This process called protein transduction greatly improves the potential of peptides to serve as therapeutics in the future.

Third, peptides open new possibilities for drug design. The small size of peptides and their presentation in a preferred conformation from a structurally resolved scaffold (e.g. Trx A) should be helpful for the identification of their bioactive structures. This is a prerequisite for using these molecules as lead structures for the design of peptide-mimetic structures.

In a preferred embodiment of the present invention the affinity domain is a peptide aptamer which binds specifically to the epidermal growth factor receptor.

A second essential component of the targeted protein degradation vehicle of the present invention is a delivery domain which delivers the functional domain into the target cell. As used herein, the term "delivery domain" denotes a peptide or polypeptide sequence which possesses the ability to traverse biological membranes. Preferably, the process occurs in a transporter- and receptor-independent fashion.

### Delivery of therapeutic proteins into mammalian cells by protein transduction

Because the cell membrane is impermeable for most peptides and proteins, the introduction of therapeutic proteins into tissues or organs proved to be rather difficult and is limited by the size and biochemical properties of the protein to be delivered. Until recently, it was not possible to introduce proteins into cells unless they were bioactive peptides of small size and highly lipophilic. In cell culture it has been possible to express exogenous proteins upon DNA-transfection, a method which is limited in efficiency and expression levels and hardly applicable *in vivo.*

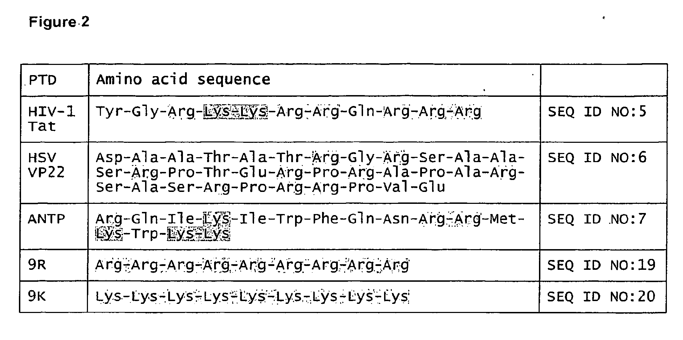

In 1988 two groups discovered that the HIV TAT protein is capable to permeate the cell membrane (Green and Loewenstein, 1988, Cell, (55) 1179-88.) (Frankel and Pabo, 1988, Cell, (55) 1189-93.). This observation has been utilized in 1994 by Fasell *et al*. to introduce a protein into cells which was linked to a 38 amino acid fragment of the HIV TAT protein (Fawell, et al., 1994, Proc Natl Acad Sci U S A, (91) 664-8.). Other protein transduction domains have been identified. Examples are the proteine transduction domains present in the Antennapedia protein of Drosophila (Joliot, et al., 1991, Proc Natl Acad Sci U S A, (88) 1864-8.) and the HSV VP22 Protein of Herpes Simplex Virus (Elliott and O'Hare, 1997, Cell, (88) 223-33.). Preferred amino acid sequences of proteins which are capable to permeate the cell membrane are shown in Fig. 2.

All protein transduction domains (PTD) are characterized by numerous basic amino acids, arginines and lysines, which are responsible for the interaction with the lipid bilayer.

In addition to the naturally occuring PTDs, there are numerous attemps to synthesize sequences which optimize protein transduction characteristics. Wender *et al*. showed that a peptide consisting of nine L-arginines is about twenty times more efficient than the naturally occuring HIV TAT domain and a D-arginine oligomer transduces proteins 100 times better into Jurkat cells. Mechanistic considerations have proposed that the guanidinium part of arginine is more important for the function than the charge or the backbone structure (Wender, et al., 2000, Proc Natl Acad Sci U S A, (97) 13003-13008). Mai et al. could show that efficiency of transduction is very much dependent on the cell line utilized and demonstrated at the same time that poly-lysine sequences are more efficient transduction domains than the TAT or arginine sequences (Mai, et al., 2002, J Biol Chem, (277) 30208-30218.). The mechanism at work which allows protein transduction domains to introduce proteins into cells through the membrane has not been totally clarified yet. Transduction can occur at 37°C and 4°C and is non-saturable. This led to the assumption that transduction does not imply a classical receptor-, transporter- or endocytosis-mediated mechanism (Derossi, et al., 1996, J Biol Chem, (271) 18188-93.). Tyagi et al. have proposed that heparin sulfate proteoglycanes on the cell surface might serve as receptors for proteins (Tyagi, et al., 2001, J Biol Chem, (276) 3254-61.). Cells which do not express such molecules or cells treated with the appropriate glycosamine glycane lyases are not able to take up TAT proteins or TAT protein fusions. The question concerning the exact mechanism is, however, still not perfectly answered.

In a preferred embodiment of the present invention the delivery domain comprises at least one sequence as given in figure 2 or a combination of such sequences. Parts of those sequences can also be combined with poly-lysine and/or poly-arginine stretches in order to improve the efficacy of the delivery domain. Further cell-penetrating peptides can be derived from Lindgren et al. (2000) Trends Pharmacol. Sci. 21, 99-103.

There are a number of examples in which heterologous peptides, proteins or even DNA have been coupled chemically or genetically with protein transduction domains. These fusions or conjugates have been introduced into cells. It seems that there are no size limits with regard to the cargo proteins. A small 20 kDa protein has been transduced into cells and found to be immediately biological active. A protein of 120 kDa has been found to require a period of about 5 minutes before intracellular biological activity could be shown (Schwarze, et al., 1999, Science, (285) 1569-72). A fusion protein of the HIV TAT PTD and β-galactosidase has been bacterially expressed and was purified in a partially denatured state. The presence of the protein was demonstrated 15 minutes after addition to the transduced cells. Enzymatic activity, however, required a period of two hours. This has been interpreted, and the requirement of the cellular machinery, for example chaperones, has been proposed. These proteins might require refolding and the assumption of a proper conformation before they become active (Schwarze, Ho, Vocero-Akbani and Dowdy, 1999, Science, (285) 1569-72). The same protein has been injected intraperitoneally into mice and it has been found to be present in all tissues like liver, kidney, lung, heart and spleen upon injection. Injected protein even crossed the blood-brain barrier and showed enzymatic activity in brain tissue (Schwarze, Ho, Vocero-Akbani and Dowdy, 1999, Science, (285) 1569-72). In further experiments a dominant-negative version of IκB (TAT-IκB⁴⁶⁻³¹⁷) has been introduced into osteoclasts as a TAT-PTD fusion. This protein was able to inhibit osteoclast differentiation (Abu-Amer, et al., 2001, J Biol Chem, (276) 30499-503.).

Proteins which are introduced into cells via protein transduction domains can exhibit therapeutic effects. A TAT-Bcl-xL fusion protein has been introduced into primary cultures of neurones and in mouse models. Mice with cerebral ischemia were intraperitoneally injected with the fusion protein and a decrease in the extent of apoptosis in brain cells was shown when compared to untreated animals. This effect was mediated by the recombinant Bcl-xL protein (Cao, et al., 2002, J Neurosci, (22) 5423-31.). These examples show that the method of protein transduction is well suited to study signal transduction pathways through specific inhibitors, but might also serve as a therapeutic tool.

Furthermore the targeted protein degradation vehicles of the present invention comprise a functional domain which degrades selectively the target protein to which the vehicle is bound. In a preferred embodiment the targeted protein degradation vehicle has a functional domain which degrades the target protein by the ubiquitination proteasome system.

### Degradation of cellular proteins by the ubiquitination-proteasome system

Proteins are not only regulated by activation, but also via reduction of the intracellular concentration. Due to this a lot of normal proteins whose concentrations must change promptly with alterations in the state of a cell, can show a short half-life. Many of these short-lived proteins are degraded rapidly at all times, while others, most notably the cyclins, are stable until they are suddenly degraded at one particular point in the cell cycle. Most of the proteins that are degraded in the cytosol are delivered to large protein complexes called proteasomes, which are present in many copies and are dispersed throughout the cell. Each proteasome consists of a central cylinder formed from multiple distinct proteases, whose active sites are thought to face an inner chamber. Each end of the cylinder is "stoppered" by a large protein complex formed from at least 10 types of polypeptides, some of which hydrolyze ATP. These protein stoppers are thought to select the proteins for destruction by binding to them and feeding them into the inner chamber of the cylinder, where multiple proteases degrade the proteins to short peptides that are then released.

Proteasomes act on proteins that have been specifically marked for destruction by the covalent attachment of a small protein called ubiquitin. Ubiquitin exists in cells either free or covalently linked to proteins. Most ubiquinated proteins have been tagged for degradation. (Some long-lived proteins such as histones are also ubiquinated, but in these cases the function of ubiquitin is not understood.) Different ubiquitin-dependent proteolytic pathways employ structurally similar but distinct ubiquitin-conjugating enzymes that are associated with recognition subunits that direct them to proteins carrying a particular degradation signal. The conjugating enzyme adds ubiquitin to a lysine residue of a target protein and thereafter adds a series of additional ubiquitin moieties, forming a multiubiquitin chain that is thought to be recognized by a specific receptor protein in the proteasome.

Peptide aptamers were only selected due to their binding affinity to a given target. As could be shown that only a small portion of the selected aptamers exhibited an inhibitory function on the target protein, their functional properties were enhanced by adding an extra domain, namely the functional domain.

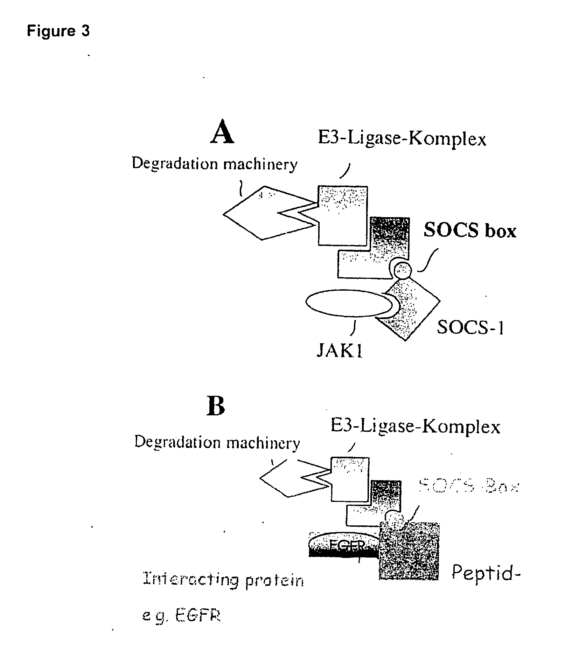

Recently domains were identified which interact specifically with the protein degradation pathway of cells. It was shown that the SOCS box domain of the SOCS-proteins (suppressors of cytokine signaling) promote the ubiquitinylation of Jak and STAT proteins (Ungureanu, et al., 2002, Mol Cell Biol, (22) 3316-26.). The function of a SOCS box is shown schematically in Fig. 3.

On one hand SOCS proteins interact via their SH2 domain with other signaling molecules such as STAT proteins and Jak molecules. On the other hand the SOCS-box interacts with E3 ubiquitin ligases (Zhang, et al., 1999, Proc Natl Acad Sci U S A, (96) 2071-6.). These ligases transfer specifically ubiquitin moieties on these molecules, which marks them for degradation in the 26 S proteasome. Therefore the SOCS box domain can be used as a functional domain to target bound molecules for degradation.

According to the present invention the targeted protein degradation vehicle has a functional domain which is selected from the group consisting of the SOCS-box, an F-box, the HECT domain and a U-box.

The SOCS-box preferably has the following amino acid sequence:
PLQELCRQRIVATVGRENLARIPLNPVLRDYLSSF (SEQ ID NO:12)
   An F-box is a protein domain of approximately 50 aa that functions as a site of protein-protein interactions. F-box proteins were first discovered as components of SCF ubiquitin-ligases complexes (named after their main components Skp1, Cullin and an F-box protein), in which they bind subtrates for ubiquitin mediated proteolysis (Kipreos and Pagano, 2000, Genome Biol, (1) ). The consensus sequence of the F-box is as follows:
KPFPLLRLPEEILRKILEKLDPIDLLRLRKVSKKWRSLVDSLNIWFKFIE (SEQ ID NO:13) (Kipreos and Pagano, 2000, Genome Biol, (1))
   HECT family domain proteins are characterized by a HECT domain (homology to E6-AP carboxyl terminus) and function as E3 ubiquitin-protein ligases, targeting specific proteins for ubiquitin-mediated proteolysis (Huibregtse, et al. 1995, PNAS, (92), 2563-2567).

The HECT domain sequence of the human E6-AP protein is as follows:

The U box is a domain of 70 amino acids present in proteins from yeast to humans. The prototype U-box protein, yeast Ufd2, was identified as a ubiquitin chain assembly factor that cooperates with a ubiquitin-activating enzyme (E1), a ubiquitin-conjugating enzyme (E2), and a ubiquitin-protein ligase (E3) to catalyze ubiquitin chain formation on artificial substrates (Hatakeyama, et al., 2001, J Biol Chem, (276) 33111-20.). As an example the U-BOX of the ubiquitin ligase UFD2a (Mus musculus) is given:

The skilled person will recognize that minor mutations, i.e. substitutions, deletions and/or additions may be made to the specific sequences referred to without changing or impairing the function of the polypeptide.

The order of the three mandatory components of the vehicle depends on the target. Usually, the affinity domain is at the N-terminus followed by the delivery domain and the functional domain at the C-terminus. Depending on the specific properties of the target this order can, however, be changed.

In addition to these three components the vehicle may also contain other parts which are helpful for the preparation and/or the function of the vehicle. An example of such additional parts are short histidine stretches which are helpful in the process of preparing the vehicle.

It is preferred that the targeted protein degradation vehicle of the invention is an isolated TPDV. An isolated TPDV is ≥ 80% pure, preferably ≥ 90% pure, more preferably ≥ 95% pure, even more preferably ≥ 99% pure and particularly preferred is a pharmaceutically pure state that is greater than 99.9% pure with respect to contaminating macromolecules, particularly other proteins and nucleic acids. It is preferred that the TPDV is free of infectious and pyrogenic agents. Preferably, a purified TPDV is substantially free of other polypeptides. When used in this context, the term "isolated" does not exclude the presence of the same polypeptide in alternative physical forms, such as dimers.

### Preferred embodiments of the present invention

A system was used to isolate specific ligands (peptide aptamers) to given target proteins. Peptides were isolated that specifically interact with the EGFR or STAT3. Fusing a SOCS-box domain, which targets the whole complex for degradation enhanced the functional interference of these aptamers with their cognate target proteins.

After adding a cell entry domain (transduction domain) to this structure a TPDV construct was generated which now comprises three functional activities in one peptide:
a) the target affinity employs specific domains to confer binding to EGF receptor as to STAT3,
b) delivery into target cells via a protein transduction domain
c) the selected degradation of proteins using specific targeting domains.

The resulting molecules are designated targeted protein degradation vehicles (abbreviated TPVDs).

In such TPDVs the domain used to target the degradation of a protein according to (c) may be SOCS-box, a F-box, a HECT domain or a U-box. The affinity domain of such a peptide may be derived from a natural interaction partner or from domains or sequences of natural interaction partners or is preferably a peptide aptamer.

Non-limiting examples of the TPDVs of the invention are polypeptides which comprise the amino acid sequences

| | |
|---|---|
| (1) | SEQ ID NO:1, SEQ ID NO:5 and SEQ ID NO:12; |
| (2) | SEQ ID NO:2, SEQ ID NO:5 and SEQ ID NO:12; |
| (3) | SEQ ID NO:3, SEQ ID NO:5 and SEQ ID NO:12; |
| (4) | SEQ ID NO:4, SEQ ID NO:5 and SEQ ID NO:12; |
| (5) | SEQ ID NO:16, SEQ ID NO:5 and SEQ ID NO:12; |
| (6) | SEQ ID NO:17, SEQ ID NO:5 and SEQ ID NO:12; |
| (7) | SEQ ID NO:18, SEQ ID NO:5 and SEQ ID NO:12; |
| | |
| (8) | SEQ ID NO:1, SEQ ID NO:6 and SEQ ID NO:12; |
| (9) | SEQ ID NO:2, SEQ ID NO:6 and SEQ ID NO: 12; |
| (10) | SEQ ID NO:3, SEQ ID NO:6 and SEQ ID NO:12; |
| (11) | SEQ ID NO:4, SEQ ID NO:6 and SEQ ID NO: 12; |
| (12) | SEQ ID NO:16, SEQ ID NO:6 and SEQ ID NO:12; |
| (13) | SEQ ID NO:17, SEQ ID NO:6 and SEQ ID NO:12; |
| (14) | SEQ ID NO:18, SEQ ID NO:6 and SEQ ID NO:12; |
| | |
| (15) | SEQ ID NO:1, SEQ ID NO:7 and SEQ ID NO:12; |
| (16) | SEQ ID NO:2, SEQ ID NO:7 and SEQ ID NO:12; |
| (17) | SEQ ID NO:3, SEQ ID NO:7 and SEQ ID NO:12; |
| (18) | SEQ ID NO:4, SEQ ID NO:7 and SEQ ID NO:12; |
| (19) | SEQ ID NO:16, SEQ ID NO:7 and SEQ ID NO:12; |
| (20) | SEQ ID NO:17, SEQ ID NO:7 and SEQ ID NO:12; |
| (21) | SEQ ID NO:18, SEQ ID NO:7 and SEQ ID NO:12; |
| | |
| (22) | SEQ ID NO:1, SEQ ID NO:19 and SEQ ID NO:12; |
| (23) | SEQ ID NO:2, SEQ ID NO:19 and SEQ ID NO:12; |
| (24) | SEQ ID NO:3, SEQ ID NO:19 and SEQ ID NO:12; |
| (25) | SEQ ID NO:4, SEQ ID NO:19 and SEQ ID NO:12; |
| (26) | SEQ ID NO:16, SEQ ID NO:19 and SEQ ID NO:12; |
| (27) | SEQ ID NO:17, SEQ ID NO:19 and SEQ ID NO:12; |
| (28) | SEQ ID NO:18, SEQ ID NO:19 and SEQ ID NO:12; |
| | |
| (29) | SEQ ID NO:1, SEQ ID NO:20 and SEQ ID NO:12; |
| (30) | SEQ ID NO:2, SEQ ID NO:20 and SEQ ID NO:12; |
| (31) | SEQ ID NO:3, SEQ ID NO:20 and SEQ ID NO:12; |
| (32) | SEQ ID NO:4, SEQ ID NO:20 and SEQ ID NO:12; |
| (33) | SEQ ID NO:16, SEQ ID NO:20 and SEQ ID NO:12; |
| (34) | SEQ ID NO:17, SEQ ID NO:20 and SEQ ID NO:12; |
| (35) | SEQ ID NO:18, SEQ ID NO:20 and SEQ ID NO:12; |
| | |
| (36) | SEQ ID NO:1, SEQ ID NO:5 and SEQ ID NO:13; |
| (37) | SEQ ID NO:2, SEQ ID NO:5 and SEQ ID NO:13; |
| (38) | SEQ ID NO:3, SEQ ID NO:5 and SEQ ID NO:13; |
| (39) | SEQ ID NO:4, SEQ ID NO:5 and SEQ ID NO:13; |
| (40) | SEQ ID NO:16, SEQ ID NO:5 and SEQ ID NO:13; |
| (41) | SEQ ID NO:17, SEQ ID NO:5 and SEQ ID NO:13; |
| (42) | SEQ ID NO:18, SEQ ID NO:5 and SEQ ID NO:13; |
| | |
| (43) | SEQ ID NO:1, SEQ ID NO:6 and SEQ ID NO:13; |
| (44) | SEQ ID NO:2, SEQ ID NO:6 and SEQ ID NO:13; |
| (45) | SEQ ID NO:3, SEQ ID NO:6 and SEQ ID NO:13; |
| (46) | SEQ ID NO:4, SEQ ID NO:6 and SEQ ID NO:13; |
| (47) | SEQ ID NO:16, SEQ ID NO:6 and SEQ ID NO:13; |
| (48) | SEQ ID NO:17, SEQ ID NO:6 and SEQ ID NO:13; |
| (49) | SEQ ID NO:18, SEQ ID NO:6 and SEQ ID NO:13; |
| | |
| (50) | SEQ ID NO:1, SEQ ID NO:7 and SEQ ID NO:13; |
| (51) | SEQ ID NO:2, SEQ ID NO:7 and SEQ ID NO:13; |
| (52) | SEQ ID NO:3, SEQ ID NO:7 and SEQ ID NO:13; |
| (53) | SEQ ID NO:4, SEQ ID NO:7 and SEQ ID NO:13; |
| (54) | SEQ ID NO:16, SEQ ID NO:7 and SEQ ID NO:13; |
| (55) | SEQ ID NO:17, SEQ ID NO:7 and SEQ ID NO:13; |
| (56) | SEQ ID NO:18, SEQ ID NO:7 and SEQ ID NO:13; |
| | |
| (57) | SEQ ID NO:1, SEQ ID NO:19 and SEQ ID NO:13; |
| (58) | SEQ ID NO:2, SEQ ID NO:19 and SEQ ID NO:13; |
| (59) | SEQ ID NO:3, SEQ ID NO:19 and SEQ ID NO:13; |
| (60) | SEQ ID NO:4, SEQ ID NO:19 and SEQ ID NO:13; |
| (61) | SEQ ID NO:16, SEQ ID NO:19 and SEQ ID NO:13; |
| (62) | SEQ ID NO:17, SEQ ID NO:19 and SEQ ID NO:13; |
| (63) | SEQ ID NO:18, SEQ ID NO:19 and SEQ ID NO:13; |
| | |
| (64) | SEQ ID NO:1, SEQ ID NO:20 and SEQ ID NO:13; |
| (65) | SEQ ID NO:2, SEQ ID NO:20 and SEQ ID NO:13; |
| (66) | SEQ ID NO:3, SEQ ID NO:20 and SEQ ID NO:13; |
| (67) | SEQ ID NO:4, SEQ ID NO:20 and SEQ ID NO:13; |
| (68) | SEQ ID NO:16, SEQ ID NO:20 and SEQ ID NO:13; |
| (69) | SEQ ID NO:17, SEQ ID NO:20 and SEQ ID NO:13; |
| (70) | SEQ ID NO:18, SEQ ID NO:20 and SEQ ID NO:13; |
| | |
| (71) | SEQ ID NO:1, SEQ ID NO:5 and SEQ ID NO:14; |
| (72) | SEQ ID NO:2, SEQ ID NO:5 and SEQ ID NO:14; |
| (73) | SEQ ID NO:3, SEQ ID NO:5 and SEQ ID NO:14; |
| (74) | SEQ ID NO:4, SEQ ID NO:5 and SEQ ID NO:14; |
| (75) | SEQ ID NO:16, SEQ ID NO:5 and SEQ ID NO:14; |
| (76) | SEQ ID NO:17, SEQ ID NO:5 and SEQ ID NO:14; |
| (77) | SEQ ID NO:18, SEQ ID NO:5 and SEQ ID NO:14; |
| | |
| (78) | SEQ ID NO:1, SEQ ID NO:6 and SEQ ID NO:14; |
| (79) | SEQ ID NO:2, SEQ ID NO:6 and SEQ ID NO:14; |
| (80) | SEQ ID NO:3, SEQ ID NO:6 and SEQ ID NO:14; |
| (81) | SEQ ID NO:4, SEQ ID NO:6 and SEQ ID NO:14; |
| (82) | SEQ ID NO:16, SEQ ID NO:6 and SEQ ID NO:14; |
| (83) | SEQ ID NO:17, SEQ ID NO:6 and SEQ ID NO:14; |
| (84) | SEQ ID NO:18, SEQ ID NO:6 and SEQ ID NO:14; |
| | |
| (85) | SEQ ID NO:1, SEQ ID NO:7 and SEQ ID NO:14; |
| (86) | SEQ ID NO:2, SEQ ID NO:7 and SEQ ID NO:14; |
| (87) | SEQ ID NO:3, SEQ ID NO:7 and SEQ ID NO:14; |
| (88) | SEQ ID NO:4, SEQ ID NO:7 and SEQ ID NO:14; |
| (89) | SEQ ID NO:16, SEQ ID NO:7 and SEQ ID NO:14; |
| (90) | SEQ ID NO:17, SEQ ID NO:7 and SEQ ID NO:14; |
| (91) | SEQ ID NO:18, SEQ ID NO:7 and SEQ ID NO:14; |
| | |
| (92) | SEQ ID NO:1, SEQ ID NO:19 and SEQ ID NO:14; |
| (93) | SEQ ID NO:2, SEQ ID NO:19 and SEQ ID NO:14; |
| (94) | SEQ ID NO:3, SEQ ID NO:19 and SEQ ID NO:14; |
| (95) | SEQ ID NO:4, SEQ ID NO:19 and SEQ ID NO:14; |
| (96) | SEQ ID NO:16, SEQ ID NO:19 and SEQ ID NO:14; |
| (97) | SEQ ID NO:17, SEQ ID NO:19 and SEQ ID NO:14; |
| (98) | SEQ ID NO:18, SEQ ID NO:19 and SEQ ID NO:14; |
| | |
| (99) | SEQ ID NO:1, SEQ ID NO:20 and SEQ ID NO:14; |
| (100) | SEQ ID NO:2, SEQ ID NO:20 and SEQ ID NO:14; |
| (101) | SEQ ID NO:3, SEQ ID NO:20 and SEQ ID NO:14; |
| (102) | SEQ ID NO:4, SEQ ID NO:20 and SEQ ID NO:14; |
| (103) | SEQ ID NO:16, SEQ ID NO:20 and SEQ ID NO:14; |
| (104) | SEQ ID NO:17, SEQ ID NO:20 and SEQ ID NO:14; |
| (105) | SEQ ID NO:18, SEQ ID NO:20 and SEQ ID NO:14; |
| | |
| (106) | SEQ ID NO:1, SEQ ID NO:5 and SEQ ID NO:15; |
| (107) | SEQ ID NO:2, SEQ ID NO:5 and SEQ ID NO:15; |
| (108) | SEQ ID NO:3, SEQ ID NO:5 and SEQ ID NO:15; |
| (109) | SEQ ID NO:4, SEQ ID NO:5 and SEQ ID NO:15; |
| (110) | SEQ ID NO:16, SEQ ID NO:5 and SEQ ID NO:15; |
| (111) | SEQ ID NO:17, SEQ ID NO:5 and SEQ ID NO:15; |
| (112) | SEQ ID NO:18, SEQ ID NO:5 and SEQ ID NO:15; |
| | |
| (113) | SEQ ID NO:1, SEQ ID NO:6 and SEQ ID NO:15; |
| (114) | SEQ ID NO:2, SEQ ID NO:6 and SEQ ID NO:15; |
| (115) | SEQ ID NO:3, SEQ ID NO:6 and SEQ ID NO:15; |
| (116) | SEQ ID NO:4, SEQ ID NO:6 and SEQ ID NO:15; |
| (117) | SEQ ID NO:16, SEQ ID NO:6 and SEQ ID NO:15; |
| (118) | SEQ ID NO:17, SEQ ID NO:6 and SEQ ID NO:15; |
| (119) | SEQ ID NO:18, SEQ ID NO:6 and SEQ ID NO:15; |
| | |
| (120) | SEQ ID NO:1, SEQ ID NO:7 and SEQ ID NO:15; |
| (121) | SEQ ID NO:2, SEQ ID NO:7 and SEQ ID NO:15; |
| (122) | SEQ ID NO:3, SEQ ID NO:7 and SEQ ID NO:15; |
| (123) | SEQ ID NO:4, SEQ ID NO:7 and SEQ ID NO:15; |
| (124) | SEQ ID NO:16, SEQ ID NO:7 and SEQ ID NO:15; |
| (125) | SEQ ID NO:17, SEQ ID NO:7 and SEQ ID NO:15; |
| (126) | SEQ ID NO:18, SEQ ID NO:7 and SEQ ID NO:15; |
| | |
| (127) | SEQ ID NO:1, SEQ ID NO:19 and SEQ ID NO:15; |
| (128) | SEQ ID NO:2, SEQ ID NO:19 and SEQ ID NO:15; |
| (129) | SEQ ID NO:3, SEQ ID NO:19 and SEQ ID NO:15; |
| (130) | SEQ ID NO:4, SEQ ID NO:19 and SEQ ID NO:15; |
| (131) | SEQ ID NO:16, SEQ ID NO:19 and SEQ ID NO:15; |
| (132) | SEQ ID NO:17, SEQ ID NO:19 and SEQ ID NO:15 or |
| (133) | SEQ ID NO:18, SEQ ID NO:19 and SEQ ID NO:15. |
| | |
| (134) | SEQ ID NO:1, SEQ ID NO:20 and SEQ ID NO:15; |
| (135) | SEQ ID NO:2, SEQ ID NO:20 and SEQ ID NO:15; |
| (136) | SEQ ID NO:3, SEQ ID NO:20 and SEQ ID NO:15; |
| (137) | SEQ ID NO:4, SEQ ID NO:20 and SEQ ID NO:15; |
| (138) | SEQ ID NO:16, SEQ ID NO:20 and SEQ ID NO:15; |
| (139) | SEQ ID NO:17, SEQ ID NO:20 and SEQ ID NO:15; or |
| (140) | SEQ ID NO:18, SEQ ID NO:20 and SEQ ID NO:15. |

A given polypeptide comprising three amino acid sequences corresponds to one of the above 140 lines, reciting three sequences. The order of the sequences in a given line is: affinity domain, delivery domain and functional domain.

The invention also relates to variants of the above polypeptides. Preferred embodiments are polypeptide variants wherein 1 to 30, 1 to 20, 1 to 15 or 1 to 10 (e.g., 1, 2, 3, 4, 5, 6, 7, 8, 9 or 10) amino acid residues of a polypeptide of the invention are substituted, added or deleted, in any combination. Particularly preferred are substitutions, additions, and deletions that do not alter the properties or activities of the polypeptide. The substitutions may be conservative amino acid substitutions, whereby a residue is substituted by another with like characteristics. Typical conservative substitutions are among Ala, Val, Leu and lie; among Ser and Thr; among the acidic residues Asp and Glu; among Asn and Gln; and among the basic residues Lys and Arg; or aromatic residues Phe and Tyr.

Another aspect of the invention is a nucleic acid coding for a targeted protein degradation vehicle according to the invention. Preferably, the nucleic acid is DNA. The invention further relates to a plasmid or vector containing such nucleic acid. The plasmid or vector may be used for transfection or viral transduction. The nucleic acid of the invention or the plasmid or vector of the invention can be used in vitro for research, diagnostic or prognostic purposes. The research, diagnostic or prognostic methods are not practised on the human or animal body, but in vitro.

Since the nucleic acid, preferably the DNA coding for the components of a targeted protein degradation vehicle is known, it is possible to express the targeted protein degradation vehicle in suitable host cells such as bacteria, in particular *E.coli* or *B.subtilis,* in yeast cells, e.g. *Saccharomyces cerevisiae* or in insect cells or eukaryotic cell cultures. The recombinantly produced targeted protein degradation vehicle can be purified by known methods, in particular affinity chromatography. Further methods of protein purification are described in Scopes R. (1994) "Protein Purification: Principles and Practice", Springer Verlag. Methods for manipulating DNA and expressing polypeptides are disclosed in Sambrook et al. (2001) "Molecular Cloning: A Laboratory Manual".

Alternatively the nucleic acid, preferably the DNA coding for a targeted protein degradation vehicle can be introduced into the cell in order to effect expression in the target cells.

By transduction of recombinant constructs having a SOCS-box and aptamers the concentration of target proteins can be efficiently reduced, which could be shown in Western blotting and immunofluorescence studies. That this process is mediated due to specific interference of the SOCS-box aptamers could be proofed by showing that ubiquitinylation of the EGFR is greatly enhanced after transduction of a SOCS-Box aptamer targeting this receptor.

The invention further relates to a medicament comprising a targeted protein degradation vehicle described herein. The medicament can be used for therapy of disorders where the selected degradation of a specific target has a beneficial effect for a patient.

In a preferred embodiment of this invention the disorder to be treated with such a TPDV can result from oncogenic proteins, due to genetical modifications, due to overexpression of the relevant protein or due to deregulation of normal proteins. The disorder is selected from the group consisting of skin cancer, melanoma, estrogen receptor-dependent and independent breast cancer, ovarian cancer, testosteron receptor-dependent and independent prostate cancer, renal cancer, colon and colorectal cancer, pancreatic cancer, bladder cancer, esophageal cancer, stomach cancer, genitourinary cancer, gastrointestinal cancer, uterine cancer, astrocytomas, gliomas, basal cancer and squameous cell carcinoma, sarcomas as Kaposi's sarcoma and osteosarcoma, head and neck cancer, small cell and non-small cell lung carcinoma, leukemia, lymphomas and other blood cell cancers.

The targeted protein degradation vehicles of the present invention can be administered to a mammalian patient to treat cancer or in any other method of the invention which involves treating a patient by any means that produces contact of the active agent with the agent's site of action in the body of the subject. Mammalian patients include humans. A TPDV of the invention can be administered by any conventional means available for use in conjunction with pharmaceuticals, either as individual therapeutic agents or in a combination of therapeutic agents. The compounds can be administered alone, but are generally administered with a pharmaceutical carrier selected on the basis of the chosen route of administration and standard pharmaceutical practice. Suitable pharmaceutical carriers are described in Remington's Pharmaceutical Sciences, Mack Publishing Company, a standard reference text in this field. The pharmaceutical compositions of the invention may be adapted for oral, parenteral, transdermal, transmucosal, rectal or intranasal administration, and may be in unit dosage form, as is well-known to those skilled in the pharmaceutical art. The term "parenteral" as used herein includes subcutaneous, intraveneous, intramuscular, or intrasternal injection or infusion techniques.

The appropriate dosage administered in any given case will of course vary depending upon known factors, such as the route of administration; the age, general health, metabolism, weight of the recipient and other factors which influence the response to the compound; the nature and extent of the symptoms; the kind of concurrent treatment; the frequency of treatment; and the effect desired.

A therapeutically effective amount of TPDV can be administered to a mammalian patient for a time and in an amount sufficient to achieve amelioration of the condition of the patient.

Figure 1 schematically shows the screening of peptide aptamers with a yeast-two-hybrid screening system. Upon interaction between bait (Gal4 DNA binding domain fused with target protein) and prey (Gal4 transactivation domain fused with thioredoxin peptide library) a complete Gal4 transcription factor is restored. This allows expression of selection marker genes, which guaranties growth under selective conditions.

Figure 2 shows the amino acid sequences of protein transduction domains (PTD) so far characterized. All PTDs contain numerous basic amino acids like arginine and lysine which mediate contact with the cell membrane. The minimal TAT transduction domain encompasses the basic residues 47 - 55, whereas residues 267 to 300 mediate transduction via the VP22 protein. In the Antennapedia protein (Antp), the third α-helix (aa 43 - 58) is responsible for transduction. Modified from (Schwarze and Dowdy, 2000, Trends Pharmacol Sci, (21) 45-8.).

Figure 3 illustrates the function of the SOCS box.

The following examples further illustrate the invention:

### Example 1

### Provision of peptide aptamer

In order to isolate molecules that specifically interact with the kinase domain of the EGFR or with the dimerization domain of *in vivo*, a peptide aptamer interaction screen was performed with the yeast strain KF1, which contains three selectable growth marker: GAL2-Ade2, GAL1-HIS3 and SPO13-URA3 (Butz, Denk, Ullmann, Scheffner and Hoppe-Seyler, 2000, Proc Natl Acad Sci U S A, (97) 6693-7).

### Methods:

The N-terminal part of the EGFR kinase domain (aa 688-621) containing the critical lysine residue, which is known to be responsible for binding of the ATP molecule, was used as bait. To identify interacting peptide aptamers for Stat3 parts of the dimerization domain, which contains the critical tyrosine 705 has been used as bait. As prey the bacterial *E.coli* trxA protein served as a scaffold to present constrained 20mer peptides of randomized sequence.

### Results:

Screening of 10⁷ yeast clones led to the isolation of three different transformants exhibiting growth in the absence of adenine. Replating showed that the clones also grew in the absence of histidine and uracil. As the clones grew under all three selection markers they can be considered as true positives and the amino acid sequences of the aptamers are presented in SEQ ID NOs:1 to 3. None of the aptamers showed any similarity with known proteins, which was expected as the aptamers are of random sequence and are not evolutionary pre-selected.

The following aptamer sequences were identified as binding specifically to the EGRFR:

| | |
|---|---|
| SEQ ID NO:1: KDI1 | VFGVSWVVGFWCQMHRRLVC |
| SEQ ID NO:2: KDI3 | TFSPVVLLYRLFLATVLIWA |
| SEQ ID NO:3: KDI4 | LSFQLSPLSLVHRWGTTVDV |

The following aptamer sequences were identified as binding specifically to STAT3:
SEQ ID NO:4 (3.40):
   SPISIPIGFWRHCALHMAVGPLSWPARVSGYSFALEVLTNF
SEQ ID NO:16 (DD-1):
   PPLVCIRSWCPLMVPHSADLGPASQWLCHRVASIALLPRYSS
SEQ ID NO:17 (DD-2):
   VGWTWMSVLVCCDGSFLVPEGPVVVQAGGAVPISGSVALMTD
SEQ ID NO:18 (DBD-1):
   PLTAVFWLIYVLAKALVTV

In order to verify the phenotype the rescued yeast expression plasmids were retransformed. All isolated peptide aptamer interacted again with the bait construct, but not with the Gal4 DNA binding domain alone. The binding affinity and specificity of the isolated peptide aptamers was verified in GST-pulidown studies.

### Example 2

### The fusion protein KDI1-9RSOCS transduces fast and efficiently into A431 cells

Peptide aptamers were selected due to their binding affinity to a given target. We could show that only a fraction of the selected aptamers exhibited an inhibitory function on the target protein. We enhanced their functional properties by adding a delivery domain and a functional domain. Protein transduction domains mediate the uptake of linked polypeptides into cells. Recently domains were identified which interact specifically with the protein degradation pathway of cells. It was shown that the SOCS box domain of the SOCS-proteins (suppressors of cytokine signaling) promote the ubiquitinylation of Jak and STAT proteins (Ungureanu, et al., 2002, Mol Cell Biol, (22) 3316-26.). The isolated aptamers were fused with this domain. The resulting proteins (named TPDVs) promote interaction with the target proteins (STAT3 and EGFR, respectively) and induce their degradation.

### Methods:

A tri-partite fusion protein was designed consisting substantially of one of the selected aptamers (affinity domain), a nine arginine protein transduction domain (delivery domain) and a SOCS box domain of the human SOCS1 protein (functional domain). The DNA constructs were cloned into the bacterial expression vector pET30. The following constructs having the indicated coding sequences were prepared:

| | |
|---|---|
| pET KDI1-9RSOCS | SEQ ID NO:8 |
| pET 30 KDI3-9RSOCS | SEQ ID NO:9 |
| pET30 KDI4-9RSOCS | SEQ ID NO:10 |
| pET 30 STAT3.40-9RSOCS | SEQ ID NO:11 |
| pET 30 DD-1-9RSOCS | SEQ ID NO:21 |
| pET 30 DD-2-9RSOCS | SEQ ID NO:22 |
| pET 30 DBD-1-9RSOCS | SEQ ID NO:23 |

The fusion proteins were bacterially expressed and purified via affinity chromatography. A431 cells (human vulval carcinoma cells) were treated for 4h with 0,5 and 1 µM KDI1-9R fusion protein. The uptake of the recombinant protein into target cells was monitored in an immunofluorescence by staining with rabbit anti-Trx/anti-rabbit rhodamin and anti-EGFR/anti-mouse FITC antibodies.

### Results:

The tri-partite fusion protein KDI1-9RSOCS was able to transduce fast and efficiently into target cells, where it co-localizes with the target protein. This could be observed by the microscopy. These data show that the SOCS box domain does not influence the transduction or binding properties of the aptamer construct. This allows to use the protein transduction method as an efficient delivery system for the targeted degradation of target proteins.

### Example 3

### Transduction of tri-partite TPDVs reduces the concentration of intracellular target protein

The dose- and time-dependence of the TPDV mechanism was monitored in immunofluorescence studies and Western blot analysis

### Methods:

A431 cells were transduced with increasing amounts of different SOCS box fusion proteins. After 3,5h the cells were induced with 50 ng/ml EGF and synthesis of new EGFR was inhibited with 25 µg/ml cycloheximide. After another 0,5h the cells were harvested, protein extracts were prepared and the presence of the EGFR was detected in a Western blot. Equal loading was monitored by staining the membrane with Ponceau Red.

Human myeloma cells U266 were transduced for 5h with 0,5 µM of aptamer 3.40 SOCS , 3.40 without a SOCS-box domain, or left untreated. Cell lysates were prepared and subjected to Western blotting with a STAT3 specific antibody. Equal loading was monitored by staining the membrane with Ponceau Red.

### Results:

With increasing amounts of fusion protein KDI1-9R the receptor concentration could be efficiently reduced. In addition a fusion protein comprising an aptamer directed against STAT3 (3.40-9RSOCS) reduced the intracellular STAT3 concentration. However, the same fusion protein without a SOCS-box-domain did not exhibit such an effect:

### Example 4:

Transduction of a tripartite SOCS-box fusion protein enhances the internalization and subsequent degradation of a target protein

In order to show the SOCS-box mediated effect in target cells, the concentration and localization of the target protein EGFR was monitored in immunofluorescence studies for different time points after transduction of the fusion protein.

### Methods:

Renca EGFR cells (murine renal carcinoma cells, stably transfected with the human EGFR) were grown on cover slips. After adhesion the cells were treated for 1, 3 or 5h with 1,5 or 3 µM of KDI1-9RSOCS. The cells were fixed and an immunofluorescence staining of the fusion protein and the receptor was performed. The cells were incubated with a mouse-anti-EGFR and a rabbit-anti-Thioredoxin antibody. The staining was visualized by using a FITC-coupled anti-mouse antibody and a rhodamin-coupled anti-rabbit antibody. The cover slips were mounted with Mowiol on glass slides and pictures were taken with a confocal laser scanning microscope.

### Results:

Untreated cells show a strong EGFR staining at the cell membrane. The same cells show a weak red background staining of the Thioredoxin antibody. In cells treated with the TPDV a strong staining is visible due to efficient transduction of the fusion protein into these cells. After one hour of treatment the EGFR concentration at the cell membrane is unaltered, which shows that the receptor concentration is not influenced. After 3h exposure to 1,5 µM KDI1-9RSOCS a punctuated EGFR staining is visible inside the cytoplasm. In addition the receptor staining is reduced. After five hours the punctuated EGFR staining disappears. The receptor levels are strongly reduced . With 3 µM of the fusion protein this effect is even more pronounced. This data shows that a recombinant TPDV protein is able to induce receptor internalization and degradation within the cytoplasm. These results are consistent with data from Western blotting studies, where 3 µM KDI1-9RSOCS causes the complete degradation of the EGFR.

### Example 5

Time and dose dependence of the SOCS-box induced degradation of the EGFR and associated proteins

In order to determine the exact kinetics of the aptamer action, STAT3 and EGFR levels are determined after exposure of cells to TPDV for different times and at different concentrations.

### Methods:

Renca EGFR were treated with 3 and 5 µM of KDI1-9RSOCS. Total cell lysates were resolved in a SDS-PAGE. After Western blotting the membrane was probed with an EGFR specific antibody. The blot was stripped and incubated with a STAT3 and a STAT5 specific antibody. Equal proteins levels in each sample and the specificity of the process was monitored by applying the cell lysates to a 12%SDS-Gel. After Western blotting the membrane was incubated with an anti-actin antibody Renca EGFR cells were transduced with 5 µM KDI1-9RSOCS. After the indicated time points, the cells were harvested and protein extracts were subjected to SDS-Page and subsequent Western blotting. The amount of the receptor was detected with an anti-EGFR antibody. The blot was normalized by staining the membrane with Ponceau Red .

### Results:

Complete degradation of the EGFR can be achieved with 3 µM KDI1-9RSOCS within 4h. Signal transduction is mediated via large protein complex - signalosomes. In an un-induced state the EGFR is already pre-associated with signaling proteins, e.g. in A431 cells Jak2, STAT1,3 and 5 are constitutively complexed with the EGFR (Olayioye, et al., 1999, J Biol Chem, (274) 17209-18). We show that this complex can be specifically targeted for degradation with a SOCS-box aptamer directed against one of the complex components. STAT3 and 5 are efficiently degraded together with the EGFR while the control protein, the cytoskeleton protein actin is completely unaffected.

### Example 6

Transduction of KDI1-9R SOCS enhances the ubiquitinylation of the EGFR

It was shown that binding of SOCS proteins induces ubiquitinylation of the target protein (Ungureanu, Saharinen, Junttila, Hilton and Silvennoinen, 2002, Mol Cell Biol, (22) 3316-26.). After dimerization the EGF receptor is internalized into early endosomes. Depending on the activation and/or ubiquitination status of the receptor the endosomes are either recycled back to the membrane or fuse with lysosomes, where the receptor is degraded. As receptor degradation is clearly dependant on the ubiquitination status of the receptor, it was assayed whether binding of the aptamer to the receptor could enhance the ubiquitinylation of the target protein.

### Methods:

A431 cells were transduced for 4h with increasing concentrations of KDI1-9R SOCS. 2h later the proteasom was inhibited by the addition of 10 µM MG132. After another 2h cell lysates were prepared and the receptor was precipitated with an EGFR specific antibody. Immunocomplexes were resolved in a SDS-PAGE. Ubiquitinylation of the receptor was detected in a Western blot with an ubiquitin specific antibody.

### Results:

We found that binding of KDI1-9RSOCS enhances ubiquitinylation of the EGFR. With 2 µM of the TPDV the receptor could be completely degraded. In the presence of the proteasomal inhibitor MG132 this degradation could be inhibited to a certain level. In the absence of a SOCS-box TPDV the receptor shows a basal mono-ubiquitinylation which is mediated via c-Cbl representing the continuous turnover of the receptor (Waterman and Yarden, 2001, FEBS Lett, (490) 142-52.). With increasing amounts of TPDV higher ubiquitinated forms of the receptor can be detected, which represent poly-ubiquitinated forms of the EGFR. Especially with 5 µM KDI1-9R SOCS the receptor becomes highly ubiquitinated. These results allow the conclusion, that due to binding of the tri-partite TPDV to the receptor, it becomes ubiquitinated which marks the receptor for degradation.

### Abbreviations:

| | |
|---|---|
| aa | amino acids |
| bp | base pair |
| *E. coli* | *Escherichia coli* |
| EGF | Epidermal growth factor |
| EGFR | Epidermal growth factor receptor |
| ErbB | Erythroblastosis retrovirus |
| FCS | Fetal calf serum |
| FITC | Fluoresceinisothiocyanat |
| h | hour |
| Herc | Human EGFR cells |
| Jak | Janus Kinase |
| KD | Kinasedomäne |
| kDa | Kilodalton |
| m | mouse |
| M | Molar |
| mM | Millimolar |
| NDF | Neu differentiation factor |
| NGF | Neuronal growth factor |
| NRG | Neuregulin |
| p | Plasmid |
| PAGE | Polyacrylamidgelelektrophorese |
| PY | Phosphotyrosin |
| r | rabbit |
| PTD | protein transductions domain |
| RTK | Rezeptor tyrosine kinase |
| *S. cervisiae* | *Saccharomyces cerevisiae* |
| SOCS | suppressors of cytokine signalling |
| STAT | Signal transducers and activators of transcription |
| Trx | Thioredoxin |
| TPDV | targeted protein degradation vehicle |
| WB | Western Blot |

## Claims

1. A targeted protein degradation vehicle comprising at least the following components:
a) a peptide aptamer which specifically binds to the epidermal growth factor receptor or to the STAT3 protein,
b) a delivery domain which delivers the vehicle into a cell, wherein the delivery domain is selected from the group consisting of the HIV TAT protein, the HSV VP22 protein, the Antennapedia protein or parts thereof or an artificial protein transduction domain such as poly-lysine or poly arginine stretches or any combination of such a transduction domain; and
c) a functional domain which degrades selectively a target protein, wherein the functional domain is selected from the group consisting of a SOCS-box, a F-box, a HECT domain and a U-box.

2. A targeted protein degradation vehicle according to claim 1 wherein the peptide aptamer specifically binds to the epidermal growth factor receptor.

3. A targeted protein degradation vehicle according to claim 2 wherein the peptide aptamer comprises an amino acid sequence selected from the sequences as shown in SEQ ID NO:1, SEQ ID NO:2 and SEQ ID NO:3.

4. A targeted protein degradation vehicle according to claim 1 wherein the peptide aptamer specifically binds to the STAT3 protein.

5. A targeted protein degradation vehicle according to claim 4 wherein the peptide aptamer comprises an amino acid sequence selected from the group consisting of the sequences SEQ ID NO:4, SEQ ID NO:16, SEQ ID NO:17 and SEQ ID NO:18.

6. A nucleic acid coding for a targeted protein degradation vehicle according to any one of claims 1-5.

7. A nucleic acid according to claim 6 wherein the nucleic acid is deoxyribonucleic acid.

8. A plasmid or vector containing a nucleic acid according to claim 6 or 7 for transfection or viral transduction.

9. In vitro-use of a nucleic acid according to claim 6 or 7 or of a plasmid or vector according to claim 8 for research, diagnostic or prognostic purposes.

10. A medicament **characterized in that** it comprises a targeted protein degradation vehicle according to any one of claims 1-5.

11. A medicament **characterized in that** it comprises a nucleic acid according to claim 6 or 7 or a plasmid or a vector according to claim 8.

12. Use of a targeted protein degradation vehicle according to anyone of claims 1-5 or of a nucleic acid according to claim 6 or 7 or of a plasmid or vector according to claim 8 for the preparation of a medicament for the treatment of a disease selected from the group consisting of skin cancer, melanoma, estrogen receptor-dependent and independent breast cancer, ovarian cancer, testosteron receptor-dependent and independent prostate cancer, renal cancer, colon and colorectal cancer, pancreatic cancer, bladder cancer, esophageal cancer, stomach cancer, genitourinary cancer, gastrointestinal cancer, uterine cancer, astrocytomas, gliomas, basal cancer and squamous cell carcinoma, sarcomas as Kaposi's sarcoma and osteosarcoma, head and neck cancer, small cell and non-small cell lung carcinoma, leukemia, lymphomas and other blood cell cancers.

## Patentansprüche

1. Targetproteinabbauvehikel, umfassend mindestens die folgenden Komponenten:
a) ein Peptidaptamer, das spezifisch an den epidermalen Wachstumsfaktor-Rezeptor oder an das STAT3-Protein bindet,
b) eine Transportdomäne, die das Vehikel in eine Zelle transportiert, wobei die Transportdomäne ausgewählt ist aus der Gruppe, bestehend aus dem HIV TAT-Protein, dem HSV VP22-Protein, dem Antennapedia-Protein oder Teilen davon oder einer künstlichen Proteintransduktionsdomäne, wie Polylysin- oder Polyargininabschnitte oder einer Kombination aus einer solchen Transduktionsdomäne; und
c) eine funktionelle Domäne, die selektiv ein Targetprotein abbaut, wobei die funktionelle Domäne ausgewählt ist aus der Gruppe, bestehend aus einer SOCS-Box, einer F-Box, einer HECT-Domäne und einer U-Box.

2. Targetproteinabbauvehikel nach Anspruch 1, wobei das Peptidaptamer spezifisch an den epidermalen Wachstumsfaktor-Rezeptor bindet.

3. Targetproteinabbauvehikel nach Anspruch 2, wobei das Peptidaptamer eine Aminosäuresequenz, ausgewählt aus den in SEQ ID NR.: 1, SEQ ID NR.: 2 und SEQ ID NR.: 3 gezeigten Sequenzen, umfasst.

4. Targetproteinabbauvehikel nach Anspruch 1, wobei das Peptidaptamer spezifisch an das STAT3-Protein bindet.

5. Targetproteinabbauvehikel nach Anspruch 4, wobei das Peptidaptamer eine Aminosäuresequenz, ausgewählt aus der Gruppe, bestehend aus den Sequenzen SEQ ID NR.: 4, SEQ ID NR.: 16, SEQ ID Nr.: 17 und SEQ ID NR.: 18, umfasst.

6. Nukleinsäure, die für ein Targetproteinabbauvehikel nach einem der Ansprüche 1 - 5 kodiert.

7. Nukleinsäure nach Anspruch 6, wobei die Nukleinsäure Desoxyribonukleinsäure ist.

8. Plasmid oder Vektor, enthaltend eine Nukleinsäure nach Anspruch 6 oder 7, zur Transfektion oder viralen Transduktion.

9. In-vitro-Verwendung einer Nukleinsäure nach Anspruch 6 oder 7 oder eines Plasmids oder Vektors nach Anspruch 8 zu Forschungs-, Diagnose- oder Prognosezwecken.

10. Medikament, **dadurch gekennzeichnet, dass** es ein Targetproteinabbauvehikel nach einem der Ansprüche 1 - 5 umfasst.

11. Medikament, **dadurch gekennzeichnet, dass** es eine Nukleinsäure nach Anspruch 6 oder 7 oder ein Plasmid oder einen Vektor nach Anspruch 8 umfasst.

12. Verwendung eines Targetproteinabbauvehikels nach einem der Ansprüche 1 - 5 oder einer Nukleinsäure nach Anspruch 6 oder 7 oder eines Plasmids oder Vektors nach Anspruch 8 zur Herstellung eines Medikaments zur Behandlung einer Krankheit, ausgewählt aus der Gruppe, bestehend aus Hautkrebs, Melanom, Östrogenrezeptor-abhängigem und -unabhängigem Brustkrebs, Eierstockkrebs, Testosteronrezeptor-abhängigem und -unabhängigem Prostatakrebs, Nierenkrebs, Kolon- und Kolon-Rektum-Krebs, Pankreaskarzinom, Blasenkrebs, Ösophaguskarzinom, Magenkrebs, Urogenitalkrebs, Magen-Darm-Krebs, Gebärmutterkrebs, Astrozytosen, Gliomata, Basalzellkarzinom und Plattenepithelkarzinom, Sarkomata wie Kaposi-Sarkom und Osteosarkom, Kopf- und Nackenkrebs, kleinzelligem und nicht kleinzelligem Lungenkarzinom, Leukämie, Lymphomata und anderen Blutzellkrebsarten.

## Revendications

1. Véhicule de dégradation de protéines ciblées, comprenant au moins les composants suivants :
a) un aptamère peptidique qui se lie spécifiquement au récepteur de facteur de croissance épidermique ou à la protéine STAT3,
b) un domaine de libération qui libère le véhicule dans une cellule, le domaine de libération étant sélectionné dans le groupe constitué de la protéine TAT du virus VIH, la protéine VP22 HSV, la protéine Antennapedia, ou des parties de celle-ci, ou un domaine de transduction de protéines artificielles tel que des étirements de polylysine ou de poly-arginine, ou une quelconque combinaison d'un tel domaine de transduction ; et
c) un domaine fonctionnel qui dégrade de sélectivement une protéine cible, le domaine fonctionnel étant sélectionné dans le groupe comprenant une SOCS-box, une F-box, un domaine HECT et une U-box.

2. Véhicule de dégradation de protéines ciblées selon la revendication 1, dans lequel l'aptamère peptidique se lie spécifiquement au récepteur de facteur de croissance épidermique.

3. Véhicule de dégradation de protéines ciblées selon la revendication 2, dans lequel l'aptamère peptidique comprenant une séquence d'acides aminés sélectionnée parmi les séquences telles que représentées dans SEQ ID N° : 1, SEQ ID N° : 2 et SEQ ID N° : 3.

4. Véhicule de dégradation de protéines ciblées selon la revendication 1, dans lequel l'aptamère peptidique se lie spécifiquement à la protéine STAT3.

5. Véhicule de dégradation de protéines ciblées selon la revendication 4, dans lequel l'aptamère peptidique comprend une séquence d'acides aminés sélectionnée dans le groupe comprenant les séquences SEQ ID N° : 4, SEQ ID N° : 16, SEQ ID N° : 17 et SEQ ID N° : 18.

6. Acide nucléique de codage pour un véhicule de dégradation de protéines ciblées selon l'une quelconque des revendications 1 à 5.

7. Acide nucléique selon la revendication 6, dans lequel l'acide nucléique est un acide désoxyribonucléique.

8. Plasmide ou vecteur contenant un acide nucléique selon la revendication 6 ou 7, pour une transfection ou une transduction virale.

9. Utilisation in vitro d'un acide nucléique selon la revendication 6 ou 7, ou d'un plasmide ou un vecteur selon la revendication 8, à des fins de recherche, diagnostic ou pronostic.

10. Médicament **caractérisé en ce qu'**il comprend un véhicule de dégradation de protéines ciblées selon l'une quelconque des revendications 1 à 5.

11. Médicament **caractérisé en ce qu'**il comprend un acide nucléique selon la revendication 6 ou 7, ou un plasmide ou un vecteur selon la revendication 8.

12. Utilisation d'un véhicule de dégradation de protéines ciblées selon l'une quelconque des revendications 1 à 5, ou d'un acide nucléique selon la revendication 6 ou 7, ou d'un plasmide ou vecteur selon la revendication 8, pour la préparation d'un médicament pour le traitement d'une maladie sélectionnée dans le groupe constitué du cancer de la peau, du mélanome, du cancer du sein indépendant ou dépendant du récepteur d'oestrogène, du cancer des ovaires, du cancer de la prostate indépendant ou dépendant du récepteur de testostérone, du cancer du rein, du cancer du côlon et colorectal, du cancer du pancréas, du cancer de la vessie, du cancer de l'oesophage, du cancer de l'estomac, du cancer génito-urinaire, du cancer gastro-intestinal, du cancer du col de l'utérus, de l'astrocytome, des gliomes, du cancer à cellules basales et du carcinome à cellules squameuses, des sarcomes comme le sarcome de Kaposi et l'ostéosarcome, du cancer de la tête et du cou, du carcinome du poumon sans petite cellule et à petites cellules, de la leucémie, de lymphomes, et d'autres cancers de cellules sanguines.
